(19) Europäisches Patentamt
European Patent Office
Office européen des brevets

(11) Publication number: **0 276 057 B1**

(12) **EUROPEAN PATENT SPECIFICATION**

(45) Date of publication of patent specification: **09.03.94**

(51) Int. Cl.[5]: **C07D 495/04**, A61K 31/495, //(C07D495/04,335:00,239:00), (C07D495/04,333:00,239:00), (C07D495/04,339:00,239:00)

(21) Application number: **88300120.8**

(22) Date of filing: **08.01.88**

(54) **Piperazinylpyrimidines as beta-adrenergic receptor blocking agents.**

(30) Priority: **21.01.87 US 5720**
**31.08.87 US 91471**

(43) Date of publication of application:
**27.07.88 Bulletin 88/30**

(45) Publication of the grant of the patent:
**09.03.94 Bulletin 94/10**

(84) Designated Contracting States:
**AT BE CH DE ES FR GB IT LI LU NL SE**

(56) References cited:
**DE-A- 1 470 354**
**FR-M- 4 321**
**FR-M- 1 555 461**
**GB-A- 1 048 986**
**GB-A- 1 072 413**

(73) Proprietor: **MERCK & CO. INC.**
**126, East Lincoln Avenue**
**P.O. Box 2000**
**Rahway New Jersey 07065-0900(US)**

(72) Inventor: **Baldwin, John J.**
**621 Gypsy Hill Circle**
**Gwynedd Valley Pennsylvania 19437(US)**
Inventor: **Wagner, Arthur F.**
**24 Sturges Way**
**Princeton New Jersey 08540(US)**
Inventor: **Tolman, Richard L.**
**29 Upper Warren Way**
**Warren New Jersey 07060(US)**
Inventor: **Pietruszkiewicz, Adolph**
**425 South 5th Street**
**North Wales Pennsylvania 19494(US)**
Inventor: **Wu, Mu T.**
**35 Lance Drive**
**Clark New Jersey 07066(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**CHEMICAL AND PHARMACEUTICAL BULLETIN, vol. 34, no. 10, 1986, pages 4150-4165, Tokyo, JP; S. OHNO et al.: "Synthesis and hypoglycemic activity of 7,8-dihydro-6H-thiopyrano[3,2-d]pyrimidine derivatives and related compounds"**

(74) Representative: **Cole, William Gwyn et al**
**European Patent Department**
**Merck & Co., Inc.**
**Terlings Park**
**Eastwick Road**
**Harlow Essex CM20 2OR (GB)**

## Description

ABSTRACT OF THE DISCLOSURE

Certain piperazinylpyrimidines have pronounced β-adrenergic receptor blocking properties and some of the compounds are particularly useful in the treatment of elevated intraocular pressure and glaucoma.

SUMMARY OF THE INVENTION

This invention is concerned with compounds of structural formula.

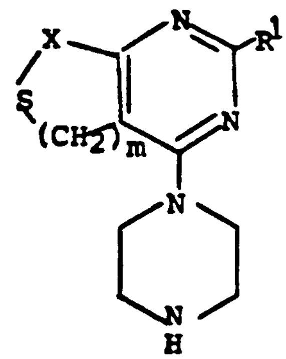

wherein X, m, and $R^1$ are as hereinafter defined which are β-adrenergic blocking agents and which are especially useful in the treatment of elevated intraocular pressure and/or glaucoma because of a low degree of blockade of non-ocular β-receptors after topical administration.

The invention is also concerned with pharmaceutical formulations of the novel compounds for use in the treatment of elevated intraocular pressure/glaucoma; and processes for preparing the compounds.

BACKGROUND OF THE INVENTION

Glaucoma is an ocular disorder associated with elevated ocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. A few β-adrenergic blocking agents are effective in reducing intraocular pressure. While many of these agents are effective in reducing intraocular pressure, they also have other characteristics, e.g. local anesthetic activity, that are not acceptable for chronic ocular use because of the potential for corneal damage, directly as the result of the anesthesia, or indirectly by the presence of foreign particles that may go unnoticed in the anesthetized eye.

Timolol, a β-adrenergic blocking agent, was found to reduce intraocular pressure and to be devoid of many unwanted side effects associated with pilocarpine and, in addition, to possess advantages over many other β-adrenergic blocking agents, e.g. to be devoid of local anesthetic properties, to have a long duration of activity, and to display minimal tolerance.

However, known β-adrenergic blocking agents have not been shown to demonstrate any meaningful oculoselectivity and, in spite of the low dose normally required for ocular administration, manifest their β-blocking properties in extra-ocular tissue, especially the pulmonary and cardiovascular systems to such an extent that they should not be administered to patients with pulmonary or cardiovascular ailments.

With this invention β-adrenergic blocking properties have been discovered in a type of chemical structure not previously known to exhibit such properties and quite different from the traditional β-blockers all or most of which have a 3-amino-2-hydroxypropoxy or similar group.

The general type of chemical structure of the novel compounds of this invention is known.

Certain 6H-7,8-dihydrothiopyrano[3,2-d]pyrimidines are disclosed in Belgian Patent 724745 as intermediates for the preparation of compounds with cardiovascular and coronary dilation activity, however, no

suggestion is made of any β-blocking activity for either the intermediates or the final products. Great Britain 2119368 discloses 6H-7,8-dihydrothiopyrano[3,2-d]pyrimidines with a very different substitution pattern on the nucleus when compared with the instant compounds. U.S. Patents 3,318,883, 3,272,811, and 3,318,881 disclose dihydrothieno[3,2-d]pyrimidines.

Chem. Pharm. Bull., 1986, 34, 4150 describes the synthesis of a series of 2-amino-7,8-dihydro-4-piperazinyl- and 4-amino-7,8-dihydro-2-piperazinyl-6H-thiopyrano[3,2-d]pyrimidines and related compounds, and the evaluation of these compounds for hypoglycemic activity.

A class of dihydrothieno[3,2-d]pyrimidine derivatives, which are stated to exhibit cardiovascular, sedative, diuretic, cytostatic, bacteriostatic, antiphlogistic and/or antipyretic actions, is described in GB-A-1072413; whilst GB-A-1048986 describes a family of dihydrothieno[3,4-d]pyrimidine derivatives which are alleged to possess cardiovascular, sedative, diuretic, analgesic or cytostatic activity.

Now, with the present invention there are provided compounds of similar structure with pronounced β-blocking properties, which are oculoselective having little or no liability by way of local anesthesia or extra-ocular β-blocking activity; pharmaceutical formulations of those compounds; the use of these compounds in the treatment of elevated intraocular pressure;

and processes for preparation of these compounds.

The principal difference between the claimed compounds and the compounds of the above references is that the claimed compounds have a hydrogen on the piperazine nitrogen and superior β-blocking activity to the reference compounds which have a substituent such as alkyl, benzyl, cyclopropyl or the like on the piperazine nitrogen, as shown in the following table:

## TABLE

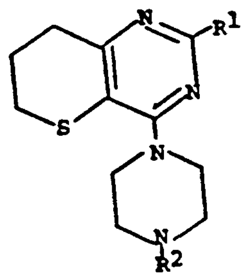

| $R^1$ | $R^2$ | Concentration (nM) To Block Binding of Ligand by 50% | |
|---|---|---|---|
| | | $\beta_1$ | $\beta_2$ |
| $-CH_3$ | H | 680 | 314 |
| 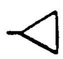 | H | 590 | 73 |
| $-CH_3$ | $-CH_2C_6H_5$ | 0%@10,000 (1) | 0%@10,000(1) |
| $-CH_3$ | $-C_2H_5$ | 3%@10,000 (1) | 27,000 |
| $-CH_3$ | 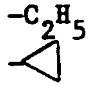 | 6%@10,000 (1) | 8%@10,000 (1) |
| $-C_4H_9$-n | $-CH_3$ | 4,300 | 4,800 |
| $-C_2H_4OH$ | $-CH_3$ | 7%@10,000 (1) | 14%@10,000 (1) |

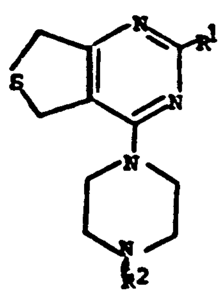

| $R^1$ | $R^2$ | $\beta_1$ | $\beta_2$ |
|---|---|---|---|
| $-CH_3$ | H | 820 | 340 |
| $-CH_3$ | $-CH_3$ | 15%@10,000 (1) | 24%@10,000 (1) |

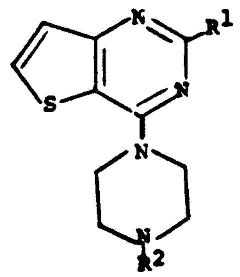

| | | | |
|---|---|---|---|
| $-C_2H_5$ | H | 300 | 140 |
| $-C_2H_5$ | $CH_3$ | 2,403 | 1,042 |

(1) % blocked at nM concentration shown; 50% blockade not achieved at reasonable concentrations.

DETAILED DESCRIPTION OF THE INVENTION

The novel compounds of this invention have structural formula:

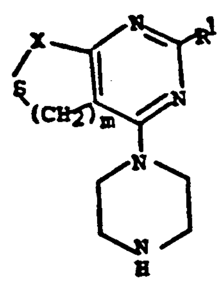

or a pharmacologically acceptable salt thereof, wherein:

X is $(CH_2)_{1-3}$ -CH=CH- OR $-CH_2SCH_2-$

m is 0 or 1; and

$R^1$ is $C_{3-6}$cycloalkyl, $C_{1-5}$alkyl or $C_{1-5}$alkylthio;

with the proviso that:

(1) when X is $-(CH_2)_3-$ and m is 0, then $R^1$ does not represent methylthio;

(2) when X is $-(CH_2)_2-$ and m is 0, then $R^1$ does not represent $C_{1-5}$alkylthio;

and

(3) when X is $-CH_2-$ and m is 1, then $R^1$ does not represent a lower alkyl group or $C_{1-5}$alkylthio.

For ophthalmic use in the treatment of elevated intraocular pressure and/or glaucoma, it is preferred that $R^1$ be cyclopropyl.

The pharmacologically acceptable salts of the compounds of this invention include those formed from inorganic acids such as hydrochloric, sulfuric and phosphoric acids and those formed from organic acids such as maleic acid, 2-naphthalenesulfonic acid, 3,5-di-tert butylsalicylic acid, 2-chloro-4,6-disulfamoylphenol, 2,5-dihydroxybenzoic acid (gentisic acid), citric acid, pamoic acid, pyruvic acid, isethionic acid, fumaric acid or the like.

Ophthalmic formulations comprising one or more of the compounds of this sub-genus of the novel compounds forms another embodiment of this invention. The ophthalmic composition of this invention may be in the form of a solution, suspensions ointment, gel, solid insert or a solution which gels on ocular administration, such as one containing a gelan gum type of polysaccharide and contains about 0.01 to 5% and especially about 0.5 to 2% by weight of medicament. Higher concentrations as, for example about 10% or lower concentration can be employed. It may be employed as the sole active ingredient or in combination with other β-blockers, such as timolol maleate; a parasympathomimetic agent such as pilocarpine, or a topically effective carbonic anhydrase inhibitor. The agents would be employed in

approximately equal amounts.

A unit dose comprises about 0.001 to 5.0 mg, preferably about 0.005 to 2.0 mg, and especially about 0.05 to 1.0 mg of active compound per eye. Multiple unit doses are administered as needed to achieve and maintain a normotensive or close to normotensive ocular condition.

The novel compounds are prepared from $R^1$-substituted-4-chloro pyrimidines IV, by treatment with piperazine or N-protected piperazine, in accordance with the following reaction scheme which also shows synthesis of compound IV.

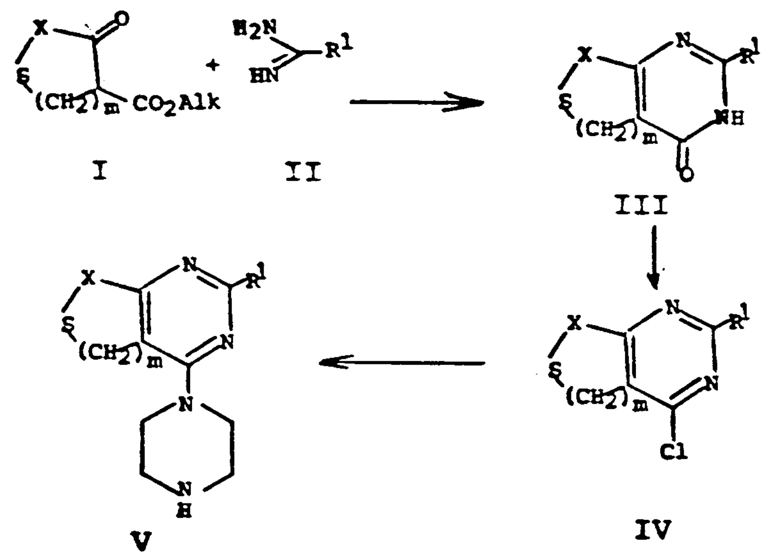

I II III IV V

wherein $R_1$ is as defined above.

In the first step of the above reaction scheme, a compound of formula I is reacted with an $R^1$-substituted amidine (II). The free base of the amidine is usually employed which is usually generated in situ by treating an amidine salt with a strong base. While any base that is a stronger base than the amidine itself may be used, generally an alkali metal base, such as sodium or potassium alkoxide is preferred. The solvent is generally a solvent compatible with the base and it is thus generally preferred to use an alcohol which corresponds to the alkoxide base used, such as methanol or ethanol. Sodium methoxide in methanol is the preferred solvent system and base. The amidine free base is then combined with compound I to prepare the 2-$R^1$-substituted-pyrimidine-4-one (III). The reaction is carried out at from 0 °C to the reflux temperature of the reaction mixture and is generally complete in from 30 minutes to 24 hours. It is preferred to carry out the reaction at about room temperature. The product is isolated using techniques known to those skilled in the art with the product generally not being purified but rather used directly in the next step.

Compound III is then reacted with a chlorinating agent such as phosphorus oxychloride, thionyl chloride or the like. While a solvent may be employed it is generally preferred to use the chlorinating agent in excess and to dispense with the use of a solvent. Generally the reaction is heated to at least 50 °C up to the reflux temperature of the reaction mixture for from about 1 hour to 3 days. It is preferred to use phosphorus oxychloride as the chlorinating agent and to heat it at about 100 °C overnight. The chlorinated compound (IV) is isolated using known techniques.

Compound V is prepared from the 4-chloro compound (IV) by displacing the chlorine with piperazine, or N-protected piperazines such as 1-piperazinecarboxaldehyde, or N-t-butoxycarbonylpiperazine.

The reaction employing piperazine is carried out preferably in an unreactive alcohol solvent although any solvent which does not react with compound IV or the amine is suitable such as ethers, THF, DMF, benzene or the like. The reaction is carried out at elevated temperatures of from 80 to 150 °C and is generally complete in from 3 to 24 hours. It is preferred to heat the reaction at from 100-120 °C in an alcohol solvent with a boiling point in excess of the reaction temperature. Thus, isoamyl alcohol with a boiling point of 132 °C is a preferred solvent.

Generally the piperazine reactant is used in excess with at least 2 and preferably 3 or more molar equivalents in order to provide a scavenger for the hydrogen chloride liberated during the course of the reaction. Alternatively, a single molar equivalent may be used along with a tertiary amine such as triethylamine or pyridine to act as the scavenger for the hydrogen chloride. The products are purified using standard techniques, and are preferably isolated as the acid addition or other physiologically acceptable

salt, such as the hydrochloride, nitrate, sulfate, maleate, citrate, and the like.

In syntheses of Compound V using 1-piperazinecarboxaldehyde, it is mixed with the appropriate 4-chloro compound (IV) in acetonitrile and refluxed for 10-24 hours, usually about 16 hours. After removal of the solvent, the residue is heated with dilute HCl at about 75 °C to reflux for about 0.5 to 2 hours.

The preparations employing N-t-butoxy-carbonylpiperazine are conducted in a high boiling alcohol such as isoamyl alcohol at about reflux temperature for about 0.5 to 2 hours. After removal of the solvent, the residue is treated at about room temperature with an acid such as trifluoroacetic acid for about 1 hour to provide the final product.

EXAMPLE 1

2-Cyclopropyl-7,8-dihydro-4-(1-piperazinyl)-6H-thiopyrano[3,2,-d]pyrimidine dihydrochloride hemihydrate

Step A: Preparation of 2-Cyclopropyl-7,8-dihydro-4-hydroxy-6H-thiopyrano[3,2-d]pyrimidine

Sodium metal (3.94 g, 0.17 g atom) was dissolved in methanol (150 ml) and cyclopropylcarboxamidine hydrochloride (21.0 g, 0.174 mol) was added followed after 1/4 hour stirring by the addition of ethyl 3-oxotetrahydrothiopyran-2-carboxylate (27.2 g, 0.145 mol). The reaction mixture was stirred at 25 °C for 18 hours then treated with ice water (200 ml) and neutralized with acetic acid to give 24.6 g of the title compound which melted at 249-250 °C.

Step B: Preparation of 2-Cyclopropyl-4-chloro-7,8-dihydro-6H-thiopyrano[3,2-d]pyrimidine

A stirred solution of product from Step A (17 g) in phosphorus oxychloride (160 ml) was heated at reflux for 1 hour. Two-thirds of the phosphorus oxychloride was distilled from the reaction mixture and the residue poured into ice water to give 12.1 g of title compound which was dried and used in Step C without further purification or characterization.

Step C: Preparation of 2-Cyclopropyl-7,8-dihydro-4-(1-piperazinyl)-6H-thiopyrano[3,2-d]pyrimidine dihydrochloride hemihydrate

A solution of product from Step B (12.1 g), and 1-piperazinecarboxaldehyde (11 ml) in acetonitrile (150 ml) was heated at reflux for 16 hours. The acetonitrile was distilled at reduced pressure and the gummy residue treated with $H_2O$ (280 ml) and concentrated hydrochloric acid (70 ml) and heated at 95 °C for 1 hour. The solvent was distilled at reduced pressure, the residue treated with ammonium hydroxide, extracted with $CH_2Cl_2$ washed with water, brine and dried over $K_2CO_3$. The $CH_2Cl_2$ was distilled at reduced pressure and the residue chromatographed on silica gel (320 g) eluting with $CHCl_3 \cdot CH_3OH$ (9:1). The pertinent fractions were combined and evaporated, the product dissolved in ethanol (60 ml), treated with 10N ethanolic HCl (10 ml) and ether (60 ml), treated with 10N ethanplic HCl (10 ml) and ether (60 ml) to give 12.5 g of title compound which melted at 205-207 °C after recrystallization from ethanol-ether.

| Analysis for $C_{14}H_2ON_4S \cdot 2HCl \cdot 1/2H_2O$ | | | |
|---|---|---|---|
| Calc: | C, 46.92; | H, 6.47; | N, 15.64; |
| Found: | C, 46,81; | H, 6.61; | N, 15.24. |

Employing the procedure substantially as described in Example 1, Steps A, B and C, but substituting for the cyclopropylcarboxamidine hydrochloride used in Step A thereof, an equimolecular amount of the

hydrochloride of an amidine of formula

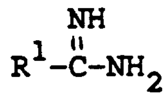

there are produced the 2-$R^1$-4-(1-piperazinyl)-7,8-dihydro-6H-thiopyrano[3,2-d]pyrimidines described in Table I in accordance with the following reaction scheme:

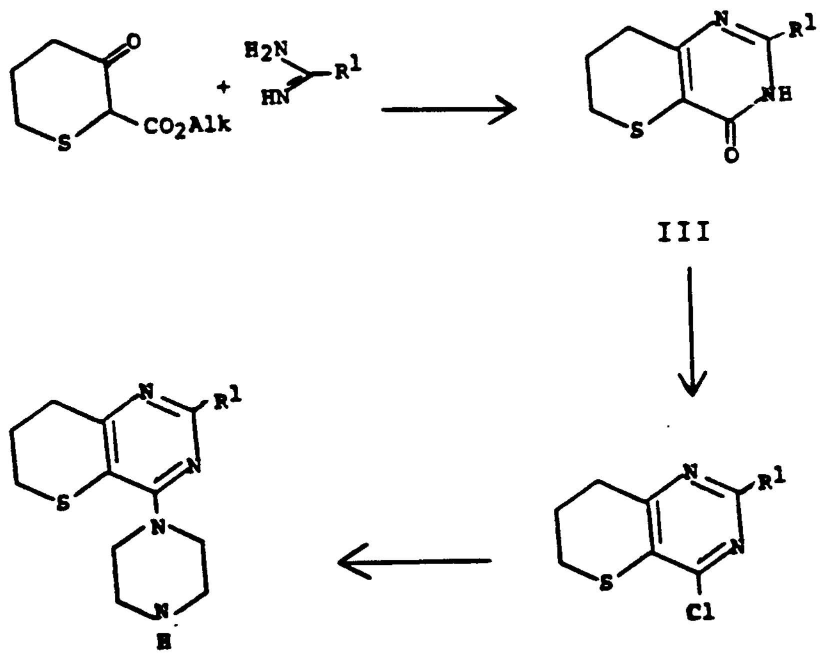

III

V

IV

TABLE I

| m.p. (°C) Compound | | | |
|---|---|---|---|
| $R^1$ | III | IV | V |
| $-CH(CH_3)_2$ | 229-231 | not purified | See Example 2 |
| $-C_2H_5$ | - | - | >275 (2 HCl" $H_2O$) |
| $-CH_3$ | - | - | *(2 HCl"9/10 $C_2H_5$ OH"1/10 $H_2O$) |
| $-CH_2CH_2CH_3$ | - | - | *(2 HCl) |

* characterized by mass spec. and n.m.r.

EXAMPLE 2

7,8-Dihydro-2-(1-methylethyl)-4-(1-piperazinyl)-6H-thiopyrano[3,2-d]pyrimidine dihydrochloride hemisemihydrate

A solution of 4-chloro-7,8-dihydro-2-(1-methylethyl)-6H-thiopyrano[3,2-d]-pyrimidine (from Table I) (2.8 g) and N-tert-butoxycarbonylpiperazine (5.0 g) in isoamyl alcohol (50 ml) was heated at reflux for 1 hour, cooled, filtered and the solvent distilled at reduced pressure. The residue was dissolved in ether, washed with $H_2O$ and brine, dried over magnesium sulfate and evaporated in vacuo. To the residue was added

trifluoroacetic acid (40 ml) which after 1 hour was distilled at reduced pressure. The residue was treated with ice and excess 10N sodium hydroxide, extracted into $CH_2Cl_2$, washed with water and brine and dried over $K_2CO_3$. The $CH_2Cl_2$ was distilled at reduced pressure and the residue was dissolved in ethanol (30 ml), treated with 10N ethanolic HCl (3 ml) and ether (30 ml) to give the title compound which melts at 256 to 258 °C after recrystallization from 2-propanol-ether.

| Anal. Calc'd for $C_{14}H_{22}N_4S \cdot 2HCl \cdot 1/4H_2O$ | | | |
|---|---|---|---|
| | C, 47.25; | H, 6.94; | N, 15.74; |
| Found: | C, 47.32; | H, 7.24; | N, 15.69. |

Similarly prepared were:

7,8-dihydro-2-methyl-4-(2-methyl-1-piperazinyl)-6H-thiopyrano[3,2-d]pyrimidine dihydrochloride, M/e = 264.

| Anal. Calc'd for $C_{13}H_{22}Cl_2N_4S \cdot O.26\ H_20$ (341.99): | | | | | |
|---|---|---|---|---|---|
| | H, 16.39; | C, 45.65; | H, 6.64; | Cl, 20.73; | S, 9.37. |
| Found: | N, 16.39; | C, 45.57; | H, 6.48; | Cl, 21.23; | S, 10.08; |

and

7,8-dihydro-2-n-propyl-4-(1-piperazinyl)-6H-thiopyrano[3,2-d]-pyrimidine dihydrochloride, M/e-278.

| Anal. Calc'd for $C_{14}H_{24}Cl_2N_4S$(351.33): | | | | | |
|---|---|---|---|---|---|
| | N, 15.95; | C, 47.86; | H, 6.89; | Cl, 20.18; | S, 9.13. |
| Found: | N, 15.52; | C, 47.64; | H, 6.61; | Cl, 20.08; | S, 8.82. |

EXAMPLE 3

7,8-dihydro-2-methyl-4-(1-piperazinyl)-6H-thiopyrano[3,2-d]pyrimidine dihydrochloride

A solution of 4-chloro-7,8-dihydro-2-methyl-6H-thiopyrano[3,2-d]pyrimidine in isoamyl alcohol (1 mmol/4 ml) was added dropwise over about 1 hour to 4 equivalents of piperazine in isoamyl alcohol (1 mmol/5ml) at 100 °C. After the chloroheterocycle had disappeared (tlc), about 4-18 hours, the mixture was concentrated under reduced pressure and the residue was partitioned between a chloroform-water system at pH 10-11 (NaOH). The chloroform extract was concentrated to dryness under reduced pressure. The residue was chromatographed on silica gel with 5% methanol in chloroform and was converted to the dihydrochloride which was isolated in 77% yield after recrystallization from ethanol. M/e = 250.

| Anal. Calc'd for $C_{12}H_{20}Cl_2\ N_4S \cdot 0.6\ C_2H_5OH \cdot 1.1\ H_20$ (370.74): | | | | | |
|---|---|---|---|---|---|
| | N, 15.12; | C, 42.76; | H, 7.01; | Cl, 19.12, | S, 8.65. |
| Found: | N, 15.28; | C, 42.42; | H, 6.50; | Cl, 18-84; | S, 8.41. |

EXAMPLE 4

7,8-Dihydro-2-ethyl-4-(1-piperazinyl)-6H-thiopyrano[3,2-d]pyrimidine dihydrochloride

A solution (1 mmole/3 ml) of 4-chloro-7,8-dihydro-2-ethyl-6H-thiopyrano[3,2-d]pyrimidine in benzene was mixed with a four-fold molar excess of piperazine in benzene (1 mmole/ml) and the reaction mixture was heated to reflux. Within an hour piperazine hydrochloride began to precipitate. The reaction was monitored by tlc for disappearance of starting chloroheterocycle. After the chloroheterocycle was consumed, the reaction mixture was filtered and the benzene phase was concentrated to dryness. The residue was taken up in a chloroform-water system in which the pH was adjusted to 10-11 with sodium hydroxide.

The chloroform extract was washed with saturated brine, dried over anhydrous magnesium sulfate and concentrated to dryness under reduced pressure. The product was converted to the dihydrochloride with a slight excess of 2N hydrochloric acid or anhydrous hydrogen chloride in ethanol. The salt which crystallized from ethanol was obtained in 45% yield.

| Anal. Calc'd for $C_{13}H_{22}Cl_2H_4S \cdot 0.2\ C_2\ H_5OH \cdot 0.25\ H_2O$ (351.03): | | | | |
|---|---|---|---|---|
| | N, 15.96; | C, 45.85; | H, 6.81; | Cl, 20.20. |
| Found: | N, 16.19; | C, 45.48; | H, 6.77; | Cl, 19.77. |

EXAMPLE 5

2-Ethyl-4-(1-piperazinyl)thieno[3,2-d]pyrimidine dihydrochloride

**Step A: Preparation of 2-Ethyl-4-hydroxythieno[3,2-d] pyrimidine**

Methyl 3-amino-2-thiophenecarboxylate (4.67 g,) and propionamidine (3 g,) were combined and heated in an oil both for 1/2 hour at 60°C; 1/2 hour at 80°C; 1/2 hour at 100°C and finally for 4 hours at 120°C. The mixture was cooled, triturated with ether, filtered and washed with ether to obtain 0.67 g of the product; m.p. 233-237°C.

**Step B: Preparation of 4-Chloro-2-ethylthieno[3,2-d] pyrimidine**

2-Ethyl-4-hydroxythieno[3,2-d]pyrimidine (0.6725 g, 0.00373 M) was refluxed with phosphorus oxychloride (20 ml) for 2 hours. The reaction mixture was cooled, concentrated under vacuum, the residue dissolved in methylene chloride, poured over ice and water and basified with ammonia. The layers were separated and the aqueous layer extracted with ether. The combined organic extracts were washed with brine, dried ($MgSO_4$) and concentrated under vacuum to obtain the product, 0.65 g, as a tan solid, m.p. 46-48°C the structure of which was confirmed by NMR.

**Step C: Preparation of 2-Ethyl-4-(1-piperazinyl)-thieno[3,2-d]pyrimidine dihydrochloride**

4-Chloro-2-ethylthieno[3,2-d]pyrimidine (0.65 g, 0.00327 M) was refluxed with 1-t-butoxycarbonylpiperazine (1.83 g, 0.0098 M) in isoamyl alcohol (12 ml) for 2 hours. The reaction mixture was cooled, diluted with 150 ml of equal volumes of ether and hexane, filtered and the filtrate concentrated under vacuum. The residue was chromatographed on silica eluting with ethyl acetate-hexane 1:1. The product fractions on concentration gave a colorless solid, 1.04 g, m.p. 117-119°C which was added to trifluoroacetic acid, 20 ml at 25°C. After stirring 1 1/2 hours, the mixture was concentrated and the residue was taken up in dilute sodium hydroxide and ether. The layers were separated, the aqueous layer extracted with methylene chloride; the combined organic extracts were washed with brine, dried ($MgSO_4$) and concentrated. The residue was chromatographed on silica eluting with THF/$NH_4OH$ (40/l). The product fractions were concentrated under vacuum to obtain the free base of the product, 0.66 g On acidification of an ethereal solution with ethanolic HCl, the dihydrochloride separated, m.p. 290-295°C.

Employing the procedure substantially as described in Example 5, Steps A to C, but substituting for the propionamidine used in Step A thereof an amidine of formula $R^1C$ (=NH) $NH_2$ described in Table II, there are produced the 2-$R^1$-4-(1-piperazinyl)thieno[3,2,-d]pyrimidines, also described in Table II.

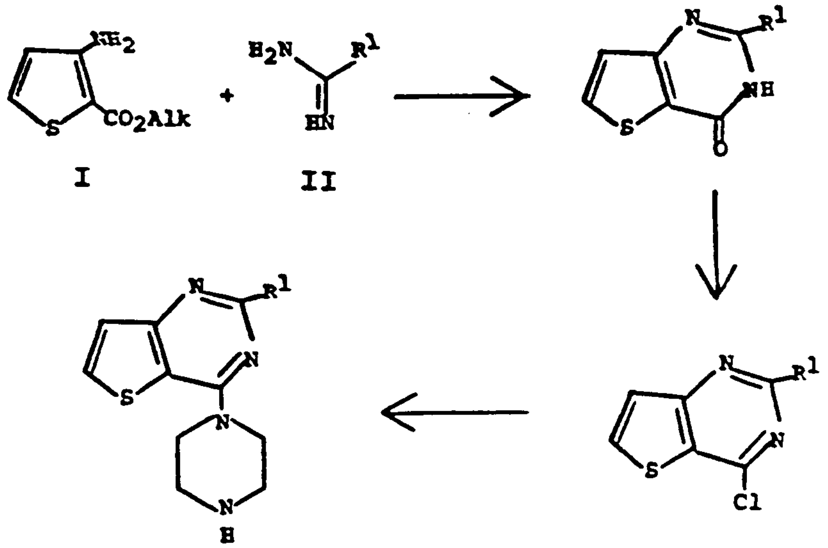

I II III IV V

TABLE II

| | m.p. (°C) Compound | | |
|---|---|---|---|
| $R^1$ | III | IV | V |
| 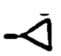 | 258-260 | 62-64 | 240-244 (d) (2 HCl) |

(1) $2HCl \cdot 0.5H_2O \cdot 0.1C_2H_5OH \cdot 0.1\ (C_2H_5)_2O$ solvate.

EXAMPLE 6

2-Ethyl-4-(1-piperazinyl)dihydrothieno[3,2-d] pyrimidine dihydrochloride

Step A: Preparation of 2-Ethyl-4-hydroxy-dihydro-thieno [3,2-d]pyrimidine

Propionamidine hydrochloride (7.60 g, 0.07 M) was added to a solution of sodium methoxide (7.56 g, 0.14 M) in methanol (35 ml) at 25 °C. The suspension was cooled with stirring to 3 °C and a mixture of methyl 3-ketotetrahydrothiophene-2-carboxylate and methyl 4-ketotetrahydrothiophene-3-carboxylate (11.21 g, 0.07 M) (JACS, 68, 2229) in methanol (15 ml) was added over 15 minutes at 3 to 6 °C. The mixture was stirred at 5 °C for 1 hour, then at 25 °C for 1 1/2 hour and finally refluxed for 3 hours and cooled. Acetic

acid (10 ml) was added and the suspension was concentrated under vacuum. The residue was triturated with water (50 ml) and the solid was filtered and washed on the funnel with water (3 x 15 ml), then ether.

The aqueous filtrate was extracted with four 75 ml portions of chloroform. The organic extracts were washed once with brine, dried ($MgSO_4$) and concentrated under vacuum. The residual oil was diluted with ether (15 ml) and hexane (15 ml). The solid that crystallized was filtered, washed with four 3 ml portions of ether and combined with the solid obtained earlier from the aqueous solution. The 2.3 g of colorless solid was shown by NMR in DMSO to be a 1:1 mixture of 2-ethyl-4-hydroxy-dihydrothieno-[3,2-d] pyrimidine and 2-ethyl-4-hydroxy-dihydrothieno[3,4-d]pyrimidine.

## Step B: Preparation of 4-Chloro-2-ethyl-dihydrothieno [3,2-d]pyrimidine

A mixture of 2.25 g of 2-ethyl-4-hydroxy-dihydrothieno[3,2-d]pyrimidine and 2-ethyl-4-hydroxy-dihydrothieno[3,4-d]pyrimidine in phosporus oxychloride (50 ml) was refluxed for 2 hours then cooled and concentrated under vacuum. The residue was dissolved in chloroform and poured into ice water. The mixture was basified with ammonia. The layers were separated and the aqueous phase extracted with chloroform. The combined organic extracts were washed with brine, dried ($MgSO_4$) and concentrated. The residual orange oil was chromatographed on 140 g of silica eluting with ethyl acetate/hexane (1/4). Concentration of the first product fractions gave an oil, 1.05 g, shown by NMR to be 4-chloro-2-ethyl-dihydrothieno[3,4-d]pyrimidine. Concentration of the second product fractions gave an oil, 1.05 g, shown by NMR to be 4-chloro-2-ethyl-dihydrothieno-[3,2-d] pyrimidine.

## Step C: Preparation of 2-Ethyl-4-(1-piperazinyl)-dihydrothieno[3,2-d]pyrimidine dihydrochloride

4-Chloro-2-ethyl-dihydrothieno[3,2-d]pyrimidine (0.63 g, 0.00314 M) was refluxed 1 3/4 hours with 1-t-butoxycarbonylpiperazine (2.05 g, 0.011 M) in isoamyl alcohol (15 ml). The mixture was concentrated under vacuum; the residue was taken up in chloroform and washed with dilute potassium carbonate and water. The organic solution was dried ($MgSO_4$) and concentrated. The residue was chromatographed on silica eluting with ethyl acetate/hexane 1:1. The product fractions were concentrated to obtain 1.0 g of a colorless solid; m.p. 92-94 °C. This intermediate was stirred in trifluoroacetic acid (15 ml) for 1 hour and then the mixture was concentrated. The residue was taken up in water, basified with potassium carbonate and extracted with chloroform. The organic extracts were washed with water, dried ($MgSO_4$) and concentrated. The residual solid, 0.67 g, was dissolved in ethanol, filtered, diluted with ether and acidified with ethanolic HCl. The solid that deposited was filtered, washed with ether and dried at 100 °C under vacuum to obtain the product salt; m.p. 210 ° (d).

By similar procedures there was prepared 2-ethylthio-4-(1-piperazinyl) dihydrothieno [3,2-d]pyrimidine, m.p. 243 °C (dec.).

EXAMPLE 7

2-Cyclopropyl-4-(1-piperazinyl) dihydrothieno[3,4-d] pyrimidine dihydrochloride

## Step A: Preparation of 2-Cyclopropyl-4-hydroxy dihydrothieno[3,4-d]pyrimidine

Cyclopropylcarboxamidine hydrochloride (9.04 g, 0.075 M) was added to a solution of sodium methoxide (4.05 g, 0.075 M) in methanol (40 ml) at 15 °C with stirring. The suspension was cooled to 5 °C and methyl 4-ketotetrahydrothiophene-3-carboxylate (10.9 g, 0.068 M) in methanol (10 ml) was added over 3/4 hours. The mixture was stirred at 5 °C for 2 hours after addition and at 25 °C overnight then concentrated under vacuum. The residue was taken up in water, acidified with acetic acid (8 ml), and the

product was filtered, washed with water and dried to obtain the crude product, m.p. 243-246 °C (2.839) the structure of which was confirmed by NMR.

**Step B: Preparation of 4-Chloro-2-cyclopropyldihydrothieno[3,4-d]pyrimidine**

2-Cyclopropyl-4-hydroxydihydrothieno[3,4-d] pyrimidine (2.4 g, 0.0123 M) was refluxed in phosphorus oxychloride (40 ml) for 1 1/2 hours. The mixture was cooled, concentrated under vacuum, the residue dissolved in chloroform, poured over ice, and basified with ammonia. The layers were separated and the organic solution dried ($MgSO_4$) and concentrated. The residue, chromatographed on silica eluting with ethyl acetate/hexane, (1/6) gave 2.2 g of product, m.p. 86.5-88.5 °C the structure of which was confirmed by NMR.

**Step C: Preparation of 2-Cyclopropyl-4-(1-piperazinyl)-dihydrothieno[3,4-d]pyrimidine dihydrochloride**

4-Chloro-2-cyclopropyldihydrothieno[3,4-d] pyrimidine (1.6 g, 0.0752 M) was refluxed with 1-t-butoxycarbonylpiperazine (4.2 g, 0.0226 M) in isoamyl alcohol (30 M) for 1 3/4 hours. The mixture was cooled, concentrated and the residue dissolved in chloroform was washed with dilute sodium hydroxide, dried ($MgSO_4$) and concentrated. The residue chromatographed on silica eluting with ethyl acetate/hexane (6/10 v/v) yielded 2.5 g of solid, m.p. 121-123 °C which was then stirred in trifluoroacetic acid (30 ml) for 2 hours. The acid reaction mixture was concentrated, the residue dissolved in water, basified with sodium hydroxide and extracted with chloroform. The organic extracts were washed with water, dried ($MgSO_4$) and concentrated. The residue, (1.48 g) was dissolved in ether and the product salt obtained by acidification with ethanolic HCl and filtration, m.p. 247-249 °C (dec.).

Employing the procedures substantially as described in Example 7 but starting with the appropriate carboxamidine in place of cyclopropyl-carboxamidine, there are prepared:
2-ethylthio-4-(1-piperazinyl)dihydrothieno[3,4-d] pyrimidine maleate, m.p. 189 °C (dec.); and 2-methyl-4-(1-piperazinyl)dihydrothieno[3,4-d] pyrimidine dihydrochloride hemihydrate, m.p. 235 °C (dec).

EXAMPLE 8

**6-Ethyl-8-[1-piperazinyl]-4H-*m*-dithieno[5,4-d] pyrimidine dihydrochloride**

**Step A: Preparation of 6-Ethyl-8-hydroxy-4H- *m*-dithieno[5,4-d] pyrimidine**

Propionamidine hydrochloride (3.82 g, 0.0352 M) was added to a solution of sodium methoxide (1.9 g, 0.0352 M) in ethanol (50 ml) at 20 °C with stirring. After 5 minutes 4-ethoxycarbonyl-5-*m*-dithianone (JACS, 82, 158, 1960) (6.19 g, 0.03 M) was added. The suspension was stirred for 20 hours at 25 °C, diluted with water, acidified with acetic acid, filtered, washed with water and dried to give 5.8 g, m.p. 268-270 °C (dec.), characterized by NMR.

**Step B: Preparation of 8-Chloro-6-ethyl-4H-*m*-dithiano[5,4-d]pyrimidine**

6-Ethyl-8-hydroxy-4H-m-dithiano[5,4-d] pyrimidine (2.9 g, 0.01353 M) was refluxed with phosphorus oxychloride (60 ml) for 2 hours. The reaction mixture was cooled, concentrated under vacuum, and the residue, dissolved in chloroform, was added to ice cold aqueous potassium carbonate. The layers were separated and the organic solution was washed with dilute ammonia, brine, then dried ($MgSO_4$) and concentrated. The residue was chromatographed on silica eluting with ethyl acetate/hexane, (1/5). The product fractions were concentrated to obtain 2.7 g, m.p. 68-70 °C and characterized by NMR.

**Step C: Preparation of 6-Ethyl-8-[1-piperazinyl]-4H-m-dithiano[5,4-d] pyrimidine dihydrochloride**

8-Chloro-6-ethyl-4H-m-dithiano[5,4-d) pyrimidine (2.32 g, 0.01 M) was refluxed with 1-t-butoxycarbonylpiperazine (4.1 g, 0.022 M) in isoamyl alcohol for 2 hours. The reaction mixture was cooled, diluted with $CHCl_3$ and washed with dilute potassium carbonate, water, dried ($MgSO_4$) and concentrated under vacuum. The residual oil was chromatographed on silica eluting with ethyl acetate/hexane, (35/120) to obtain 3.5 g of the protected piperazine, m.p. 105.5-107.5 °C. The intermediate was stirred in trifluoroacetic acid (40 ml) for 1 1/2 hours at 25 °C and the mixture was then concentrated under vacuum. The residue was basified in aqueous potassium carbonate and extracted with chloroform. The organic extracts were washed with brine, dried ($MgSO_4$) and concentrated. The residue was dissolved in 60 ml of an equimolor mixture of ethanol and ether, filtered, diluted with ether, acidified with ethanolic HCl and filtered to obtain the hygroscopic product, m.p. 120-125 °C.

Using the procedures substantially as described in Example 8, but starting with cyclopropylcarboxamidine in place of propionamidine there were prepared in sequence 6-cyclopropyl-8-hydroxy-4H-m-dithiano[5,4-d]pyrimidine, m.p. 236-287 °C (dec); 8-chloro-6-cyclopropyl-4H-m-dithiano[5,4-d] pyrimidine, m.p. 78-80 °C; 6-cyclopropyl-8-(1-piperazinyl)-4H-m-dithiano[5,4-d] pyrimidine dihydrochloride, m.p. 135-140 °C (dec.).

6-Methyl-8-(1-piperazinyl)-4H-m-dithiano[5,4-d] pyrimidinedihydrochloride, m.p. 215 °C was prepared similarly.

EXAMPLE 9

| Solution Composition | |
|---|---|
| 2-Cyclopropyl-7,8-dihydro-4-(1-piperazinyl)-6H-thiopyrano[3,2-d]-pyrimidine dihydrochloride hemihydrate | 6.8 mg. |
| Sodium Chloride | 7.4 mg. |
| Benzalkonium chloride | 0.10 mg. |
| Sodium acetate anhydrous | 0.82 mg. |
| Water for injection q.s. and. | 1.0 ml. |

The active compound, salts, and benzalkonium chloride are added to and dissolved in water and the final solution diluted to volume. The solution is rendered sterile by filtration through a sterilizing filter.

EXAMPLE 10

| | |
|---|---|
| 7,8-Dihydro-2-(1-methylethyl)-4-(1-piperazinyl)-6H-thiopyrano-[3,2-d] pyrimidine dihydrochloride hemihydrate | 5 mg. |
| Petrolatum q.s. and. | 1 gram |

The active compound and the petrolatum are aseptically combined.

EXAMPLE 11

| | |
|---|---|
| 2-Cyclopropyl-7,8-dihydro-4-(1-piperazinyl)-6H-thiopyrano[3,2-d]-pyrimidine dihydrochloride hemihydrate | 1 mg. |
| Hydroxypropylcellulose q.s. | 12 mg. |

Ophthalmic inserts are manufactured from compression molded films which are prepared on a Carver Press by subjecting the powder mixture of the above ingredients to a compressional force of 12,000 lbs. (gauge) at 300 °F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30 °C) for two to four days. After removal from the humidity cabinet, the vials are stoppered and then capped. The vials containing the hydrated insert are then autoclaved at 250 °F for 1/2 hour.

**Claims**

**Claims for the following Contracting States : AT, BE, CH, DE, FR, GB, IT, LI, LU, NL, SE**

**1.** A compound of structural formula:

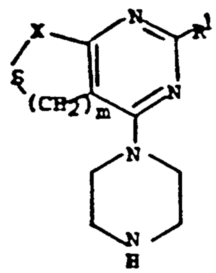

or an ophthalmologically acceptable salt thereof, wherein:

X is $(CH_2)_{1-3}$,-CH=CH- or $-CH_2SCH_2-$

m is zero or 1: and

$R^1$ is $C_{3-6}$cycloalkyl, $C_{1-5}$alkyl or $C_{1-5}$alkylthio;

with the proviso that:

(1) when X is $-(CH_2)_3-$ and m is 0, then $R^1$ does not represent methylthio;

(2) when X is $-(CH_2)_2-$ and m is 0, then $R^1$ does not represent $C_{1-5}$alkylthio;

and

(3) when X is $-CH_2-$ and m is 1, then $R^1$ does not represent a lower alkyl group or $C_{1-5}$alkylthio.

**2.** The compound of Claim 1, wherein $R^1$ is $C_{1-3}$alkyl or cyclopropyl, or an ophthalmologically acceptable salt thereof.

**3.** The compound of Claim 1, wherein X is $-(CH_2)_3-$ and m is zero, or an ophthalmologically acceptable salt thereof.

**4.** The compound which is:

2-ethyl-4-(1-piperazinyl)dihydrothieno[3,4-d] pyrimidine;
6-ethyl-8-(1-piperazinyl)-4H-m-dithiano[5,4-d] pyrimidine;
7,8-dihydro-2-(1-methylethyl)-4-(1-piperazinyl)-6H-thiopyrano[3,2-d] pyrimidine
2-ethylthio-4-(1-piperazinyl)dihydrothieno[3,2-d] pyrimidine;
2-ethyl-4-(1-piperazinyl)dihydrothieno[3,2-d] pyrimidine;
7,8-dihydro-2-methyl-4-(1-piperazinyl)-6H-thiopyrano[3,2-d]pyrimidine;
7,8-dihydro-4-(1-piperazinyl)-2-propyl-6H-thiopyrano[3,2-d]pyrimidine;
2-cyclopropyl-4-(1-piperazinyl)dihydrothieno[3,4-d] pyrimidine;
2-cyclopropyl-4-(1-piperazinyl)thieno[3,2-d] pyrimidine;

2-ethyl-4-(1-piperazinyl)thieno[3,2-d] pyrimidine;
7,8-dihydro-2-ethyl-4-(1-piperazinyl)-6H-thiopyrano [3,2-d] pyrimidine; or
2-cyclopropyl-7,8-dihydro-4-(1-piperazinyl)-6H-thiopyrano[3,2-d] pyrimidine; or a pharmaceutically acceptable salt thereof.

**5.** 2-cyclopropyl-7,8-dihydro-4-(1-piperazinyl)-6H-thiopyrano-[3,2-d]pyrimidine or a pharmaceutically acceptable salt thereof.

**6.** An ophthalmic formulation for the treatment of elevated intraocular pressure and glaucoma comprising an ophthalmologically acceptable carrier and an effective intraocular pressure lowering and antiglaucoma amount of the compound of Claim 1, or an ophthalmologically acceptable salt thereof.

**7.** The use of a compound of Claim 1 for the preparation of a medicament useful for treating elevated intraocular pressure or glaucoma.

**Claims for the following Contracting State : ES**

**1.** A process for the preparation of a compound of structural formula:

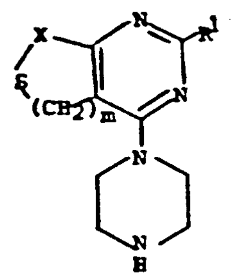

or an ophthalmologically acceptable salt thereof, wherein:
X is $(CH_2)_{1-3}$,-CH=CH- or $-CH_2SCH_2-$
m is zero or 1; and
$R^1$ is $C_{3-6}$cycloalkyl, $C_{1-5}$alkyl or $C_{1-5}$alkylthio;
with the proviso that:
(1) when X is $-(CH_2)_3-$ and m is 0, then $R^1$ does not represent methylthio;
(2) when X is $-(CH_2)_2-$ and m is 0, then $R^1$ does not represent $C_{1-5}$alkylthio;
and
(3) when X is $-CH_2-$ and m is 1, then $R^1$ does not represent a lower alkyl group or $C_{1-5}$alkylthio;
which process comprises treatment of a chloro-pyrimidine of formula IV:

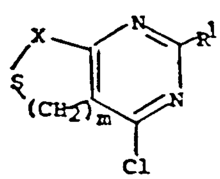

IV

with piperazine or N-protected piperazine.

**2.** The process of Claim 1, wherein $R^1$ is $C_{1-3}$alkyl or cyclopropyl, or an ophthalmologically acceptable salt thereof.

**3.** The process of Claim 1, wherein X is $-(CH_2)_3-$ and m is zero, or an ophthalmologically acceptable salt thereof.

4. The process of claim 1 for the preparation of a compound which is:
2-ethyl-4-(1-piperazinyl)dihydrothieno[3,4-d] pyrimidine;
6-ethyl-8-(1-piperazinyl)-4H-m-dithiano[5,4-d] pyrimidine;
7,8-dihydro-2-(1-methylethyl)-4-(1-piperazinyl)-6H-thiopyrano[3,2-d] pyrimidine
2-ethylthio-4-(1-piperazinyl)dihydrothieno[3,2-d] pyrimidine;
2-ethyl-4-(1-piperazinyl)dihydrothieno[3,2-d] pyrimidine;
7,8-dihydro-2-methyl-4-(1-piperazinyl)-6H-thiopyrano[3,2-d]pyrimidine;
7,8-dihydro-4-(1-piperazinyl)-2-propyl-6H-thiopyrano[3,2-d]pyrimidine;
2-cyclopropyl-4-(1-piperazinyl)dihydrothieno[3,4-d] pyrimidine;
2-cyclopropyl-4-(1-piperazinyl)thieno[3,2-d] pyrimidine;
2-ethyl-4-(1-piperazinyl)thieno[3,2-d] pyrimidine;
7,8-dihydro-2-ethyl-4-(1-piperazinyl)-6H-thiopyrano [3,2-d] pyrimidine; or
2-cyclopropyl-7,8-dihydro-4-(1-piperazinyl)-6H-thiopyrano[3,2-d] pyrimidine; or a pharmaceutically acceptable salt thereof.

5. A process as claimed in Claim 1 for the preparation of 2-cyclopropyl-7,8-dihydro-4-(1-piperazinyl)-6H-thiopyrano-[3,2-d]pyrimidine or a pharmaceutically acceptable salt thereof.

6. A process for preparing an ophthalmic formulation for the treatment of elevated intraocular pressure and glaucoma comprising mixing an ophthalmologically acceptable carrier and an effective intraocular pressure lowering and antiglaucoma amount of the compound prepared as claimed in Claim 1, or an ophthalmologically acceptable salt thereof.

**Patentansprüche**

**Patentansprüche für folgende Vertragsstaaten : AT, BE, CH, DE, FR, GB, IT, LI, LU, NL, SE**

1. Verbindung der Strukturformel

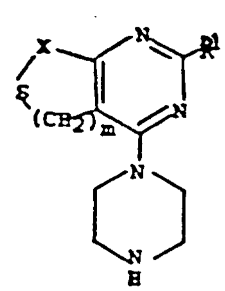

oder ein ophthalmologisch geeignetes Salz davon,
wobei
X $(CH_2)_{1-3}$, -CH = CH- oder $CH_2SCH_2$ ist,
m 0 oder 1 ist und
$R^1$ $C_3$-$C_6$-Cycloalkyl, $C_1$-$C_5$-Alkyl oder $C_1$-$C_5$-Alkylthio bedeutet,
mit der Maßgabe, daß
(1) wenn X $-(CH_2)_3-$ und m 0 ist, $R^1$ nicht Methylthio bedeutet;
(2) wenn X $-(CH_2)_2-$ und m 0 ist, $R^1$ nicht $C_1$-$C_5$-Alkylthio bedeutet und
(3) wenn X $-CH_2-$ und m 1 ist, $R^1$ nicht eine niedere Alkylgruppe oder $C_1$-$C_5$-Alkylthio bedeutet.

2. Verbindung nach Anspruch 1, wobei $R^1$ $C_1$-$C_3$-Alkyl oder Cyclopropyl bedeutet, oder ein ophthalmologisch geeignetes Salz davon.

3. Verbindung nach Anspruch 1, wobei X $-(CH_2)_3-$ und m 0 ist, oder ein ophthalmologisch geeignetes Salz davon.

4. Verbindung
2-Ethyl-4-(1-piperazinyl)dihydrothieno[3,4-d]-pyrimidin,
6-Ethyl-8-(1-piperazinyl)-4H-m-dithiano[5,4-d]-pyrimidin,
7,8-Dihydro-2-(1-methylethyl)-4-(1-piperazinyl)-6H-thiopyrano[3,2-d]-pyrimidin,

2-Ethylthio-4-(1-piperazinyl)-dihydrothieno[3,2-d]-pyrimidin,
2-Ethyl-4-(1-piperazinyl)-dihydrothieno[3,2-d]-pyrimidin,
7,8-Dihydro-2-methyl-4-(1-piperazinyl)-6H-thiopyrano[3,2-d]-pyrimidin,
7,8-Dihydro-4-(1-piperazinyl)-2-propyl-6H-thiopyrano[3,2-d]-pyrimidin,
2-Cyclopropyl-4-(1-piperazinyl)dihydrothieno[3,4-d]-pyrimidin,
2-Cyclopropyl-4-(1-piperazinyl)thieno[3,2-d]-pyrimidin,
2-Ethyl-4-(1-piperazinyl)thieno[3,2-d]-pyrimidin,
7,8-Dihydro-2-ethyl-4-(1-piperazinyl)-6H-thiopyrano[3,2-d]-pyrimidin oder
2-Cyclopropyl-7,8-dihydro-4-(1-piperazinyl)-6H-thiopyrano[3,2-d]-pyrimidin
oder ein pharmazeutisch annehmbares Salz davon.

**5.** 2-Cyclopropyl-7,8-dihydro-4-(1-piperazinyl)-6H-thiopyrano[3,2-d]-pyrimidin oder ein pharmazeutisch annehmbares Salz davon.

**6.** Ophthalmologisches Mittel zur Behandlung von erhöhtem Augeninnendruck und Glaukom, umfassend einen ophthalmologisch geeigneten Träger und eine wirksame Menge zur Verringerung des Augeninnendrucks und gegen Glaukom der Verbindung nach Anspruch 1 oder eines ophthalmologisch geeigneten Salzes davon.

**7.** Verwendung einer Verbindung nach Anspruch 1 zur Herstellung eines Arzneimittels, das zur Behandlung von erhöhtem Augeninnendruck und Glaukom geeignet ist.

**Patentansprüche für folgenden Vertragsstaat : ES**

**1.** Verfahren zur Herstellung einer Verbindung der Strukturformel

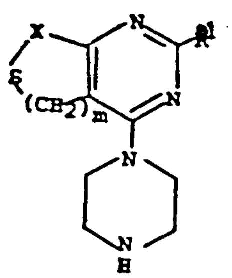

oder eines ophthalmologisch geeigneten Salzes davon,
wobei
X $(CH_2)_{1-3}$, -CH = CH- oder $CH_2SCH_2$ ist,
m 0 oder 1 ist und
$R^1$ $C_3$-$C_6$-Cycloalkyl, $C_1$-$C_5$-Alkyl oder $C_1$-$C_5$-Alkylthio bedeutet,
mit der Maßgabe, daß
(1) wenn X $-(CH_2)_3-$ und m 0 ist, $R^1$ nicht Methylthio bedeutet;
(2) wenn X $-(CH_2)_2-$ und m 0 ist, $R^1$ nicht $C_1$-$C_5$-Alkylthio bedeutet und
(3) wenn X $-CH_2-$ und m 1 ist, $R^1$ nicht eine niedere Alkylgruppe oder $C_1$-$C_5$-Alkylthio bedeutet,
wobei das Verfahren umfaßt die Behandlung eines Chlor-pyrimidins der Formel IV

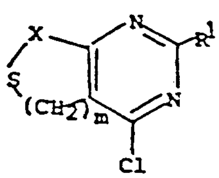

IV

mit Piperazin oder N-geschütztem Piperazin.

**2.** Verfahren nach Anspruch 1, wobei $R^1$ $C_1$-$C_3$-Alkyl oder Cyclopropyl bedeutet, oder ein ophthalmologisch geeignetes Salz davon.

3. Verfahren nach Anspruch 1, wobei X $-(CH_2)_3-$ und m 0 ist, oder ein ophthalmologisch geeignetes Salz davon.

4. Verfahren nach Anspruch 1 zur Herstellung der Verbindung
2-Ethyl-4-(1-piperazinyl)dihydrothieno[3,4-d]-pyrimidin,
6-Ethyl-8-(1-piperazinyl)-4H-m-dithiano[5,4-d]-pyrimidin,
7,8-Dihydro-2-(1-methylethyl)-4-(1-piperazinyl)-6H-thiopyrano[3,2-d]-pyrimidin,
2-Ethylthio-4-(1-piperazinyl)-dihydrothieno[3,2-d]-pyrimidin,
2-Ethyl-4-(1-piperazinyl)-dihydrothieno[3,2-d]-pyrimidin,
7,8-Dihydro-2-methyl-4-(1-piperazinyl)-6H-thiopyrano[3,2-d]-pyrimidin,
7,8-Dihydro-4-(1-piperazinyl)-2-propyl-6H-thiopyrano[3,2-d]-pyrimidin,
2-Cyclopropyl-4-(1-piperazinyl)dihydrothieno[3,4-d]-pyrimidin,
2-Cyclopropyl-4-(1-piperazinyl)thieno[3,2-d]-pyrimidin,
2-Ethyl-4-(1-piperazinyl)thieno[3,2-d]-pyrimidin,
7,8-Dihydro-2-ethyl-4-(1-piperazinyl)-6H-thiopyrano[3,2-d]-pyrimidin oder
2-Cyclopropyl-7,8-dihydro-4-(1-piperazinyl)-6H-thiopyrano[3,2-d]-pyrimidin oder eines pharmazeutisch geeigneten Salzes davon.

5. Verfahren nach Anspruch 1 zur Herstellung von 2-Cyclopropyl-7,8-dihydro-4-(1-piperazinyl)-6H-thiopyrano[3,2-d]-pyrimidin oder einem pharmazeutisch geeigneten Salz davon.

6. Verfahren zur Herstellung eines ophthalmologischen Mittels zur Behandlung von erhöhtem Augeninnendruck und Glaukom, umfassend das Vermischen eines ophthalmologisch geeigneten Trägers und einer wirksamen Menge zur Verringerung des Augeninnendrucks und gegen Glaukom der Verbindung, hergestellt nach Anspruch 1 oder eines ophthalmologisch geeigneten Salzes davon.

**Revendications**
**Revendications pour les Etats contractants suivants : AT, BE, CH, DE, FR, GB, IT, LI, LU, NL, SE**

1. Composé de formule structurelle :

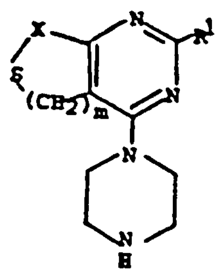

ou un de ses sels acceptables du point de vue ophthalmologique, dans laquelle :
X est $(CH_2)_{1-3}$,-CH=CH- ou $-CH_2SCH_2-$;
m est zéro ou 1 ; et
$R^1$ est un cycloalkyle en $C_3$ à $C_6$, un alkyle en $C_1$ à $C_5$ ou un alkylthio en $C_1$ à $C_5$ ;
sous la condition que :
(1) lorsque X est $-(CH_2)_3-$ et m est 0, alors $R^1$ ne représente pas du méthylthio ;
(2) lorsque X est $-(CH_2)_2-$ et m est 0, alors $R^1$ ne représente pas un alkylthio en $C_1$ à $C_5$ ; et
(3) lorsque X est $-CH_2-$ et m est 1, alors $R^1$ ne représente pas un groupe alkyle inférieur ni un alkylthio en $C_1$ à $C_5$.

2. Le composé de la revendication 1, dans lequel $R^1$ est un alkyle en $C_1$ à $C_3$ ou du cyclopropyle, ou bien un sel acceptable du point de vue ophthalmologique de celui-ci.

3. Le composé de la revendication 1, dans lequel X est $-(CH_2)_3-$ et m est zéro, ou bien un sel acceptable du point de vue ophthalmologique de celui-ci.

4. Le composé qui est :
la 2-éthyl-4-(1-pipérazinyl)dihydrothiéno[3,4-d]pyrimidine;
la 6-éthyl-8-(1-pipérazinyl)-4H-m-diothiano[5,4-d]pyrimidine ;
la 7,8-dihydro-2-(1-méthyléthyl)-4-(1-pipérazinyl)-6H-thiopyrano[3,2-d]pyrimidine ;
la 2-éthylthio-4-(1-pipérazinyl)dihydrothiéno[3,2-d]pyrimidine ;
la 2-éthyl-4-(1-pipérazinyl)dihydrothiéno[3,2-d]pyrimidine;
la 7,8-dihydro-2-méthyl-4-(1-pipérazinyl)-6H-thiopyrano[3,2-d]pyrimidine ;
la 7,8-dihydro-4-(1-pipérazinyl)-2-propyl-6H-thiopyrano[3,2-d]pyrimidine ;
la 2-cyclopropyl-4-(1-pipérazinyl)dihydrothiéno-[3,4-d]pyrimidine ;
la 2-éthyl-4-(1-pipérazinyl)-thiéno[3,2-d]pyrimidine;
la 7,8-dihydro-2-éthyl-4-(1-pipérazinyl)-6H-thiopyrano[3,2-d] pyrimidine ; ou
la 2-cyclopropyl-7,8-dihydro-4-(1-pipérazinyl)-6H-thiopyrano[3,2-d] pyrimidine ; ou un de leurs sels pharmaceutiquement acceptables.

5. La 2-cyclopropyl-7,8-dihydro-4-(1-pipérazinyl)-6H-thiopyrano-[3,2-d]pyrimidine ou un sel pharmaceutiquement acceptable de celui-ci.

6. Une formalution ophthalmique pour le traitement de la pression intraoculaire élevée et du glaucome, comportant un support acceptable du point de vue ophthalmologique et une quantité efficace pour abaisser la pression intraoculaire et antiglaucome du composé de la revendication 1, ou d'un sel acceptable du point de vue ophthalmologique de celui-ci.

7. L'utilisation d'un composé de la revendication 1, pour la préparation d'un médicament utilisable pour traiter une pression intraoculaire élevée ou le glaucome.

**Revendications pour l'Etat contractant suivant : ES**

1. Un procédé pour la préparation d'un composé de formule structurelle :

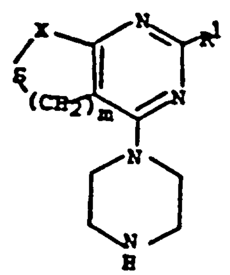

ou un de ses sels acceptables du point de vue ophthalmologique, dans laquelle :
X est $(CH_2)_{1-3}$,-CH = CH- ou $-CH_2SCH_2-$;
m est zéro ou 1 ; et
$R^1$ est un cycloalkyle en $C_3$ à $C_6$, un alkyle en $C_1$ à $C_5$ ou un alkylthio en $C_1$ à $C_5$ ;
sous la condition que :
(1) lorsque X est $-(CH_2)_3-$ et m est 0, alors $R^1$ ne représente pas du méthylthio ;
(2) lorsque X est $-(CH_2)_2-$ et m est 0, alors $R^1$ ne représente pas un alkylthio en $C_1$ à $C_5$ ; et
(3) lorsque X est $-CH_2-$ et m est 1, alors $R^1$ ne représente pas un groupe alkyle inférieur ni un alkylthio en $C_1$ à $C_5$ ;
procédé selon lequel on traite une chloro-pyrimidine de la formule IV :

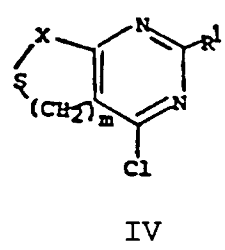

IV

avec de la pipérazine ou une pipérazine protégée en N.

2. Le procédé de la revendication 1, dans lequel $R^1$ est un alkyle en $C_1$ à $C_3$ ou du cyclopropyle, ou bien un sel acceptable du point de vue ophthalmologique de celui-ci.

3. Le procédé de la revendication 1, dans lequel X est $-(CH_2)_3-$ et m est zéro, ou bien un sel acceptable du point de vue ophthalmologique de celui-ci.

4. Le procédé de la revendication pour la préparation d'un composé qui est :
la 2-éthyl-4-(1-pipérazinyl)dihydrothiéno[3,4-d]pyrimidine;
la 6-éthyl-8-(1-pipérazinyl)-4H-m-diothiano[5,4-d]pyrimidine ;
la 7,8-dihydro-2-(1-méthyléthyl)-4-(1-pipérazinyl)-6H-thiopyrano[3,2-d]pyrimidine ;
la 2-éthylthio-4-(1-pipérazinyl)dihydrothiéno[3,2-d]pyrimidine ;
la 2-éthyl-4-(1-pipérazinyl)dihydrothiéno[3,2-d]pyrimidine;
la 7,8-dihydro-2-méthyl-4-(1-pipérazinyl)-6H-thiopyrano[3,2-d]pyrimidine ;
la 7,8-dihydro-4-(1-pipérazinyl)-2-propyl-6H-thiopyrano[3,2-d]pyrimidine ;
la 2-cyclopropyl-4-(1-pipérazinyl)dihydrothiéno-[3,4-d]pyrimidine ;
la 2-éthyl-4-(1-pipérazinyl)-thiéno[3,2-d]pyrimidine;
la 7,8-dihydro-2-&thyl-4-(1-pipérazinyl)-6H-thiopyrano[3,2-d] pyrimidine ; ou
la 2-cyclopropyl-7,8-dihydro-4-(1-pipérazinyl)-6H-thiopyrano[3,2-d] pyrimidine ; ou un de leurs sels pharmaceutiquement acceptables de celui-ci.

5. Un procédé tel que revendiqué dans la revendication 1 pour la préparation du 2-cyclopropyl-7,8-dihydro-4-(1-pipérazinyl)-6H-thiopyrano-[3,2-d]pyrimidine ou un sel pharmaceutiquement acceptable de celui-ci.

6. Un procédé pour la préparation d'une formalution ophthalmique pour le traitement de la pression intraoculaire élevée et du glaucome, comportant un support acceptable du point de vue ophthalmologique et une quantité efficace pour abaisser la pression intraoculaire, et antiglaucome du composé de la revendication 1, ou d'un sel acceptable du point de vue ophthalmologique de celui-ci.